# EUROPEAN PATENT APPLICATION

(11) **EP 3 929 297 A1**
(43) Date of publication of application: **29.12.2021**
(21) Application number: 20925003.4
(22) Date of filing: 18.12.2020
(51) Int. Cl.: C12N 15/74, C12N 1/21, C12P 13/08, C12R 1/13

(54) **RECOMBINANT MICROORGANISM FOR PRODUCING L-VALINE, CONSTRUCTION METHOD THEREFOR, AND APPLICATION THEREOF**

(30) Priority: 13.05.2020 CN 202010401422
(71) Applicant: Anhui Huaheng Biotechnology Co., Ltd., Suangfeng Industrial Park Hefei, Anhui 21131 (CN); Tianjin Institute Of Industrial Biotechnology, Chinese Academy of Sciences, Tianjin 300308 (CN)
(72) Inventor: ZHANG, Xueli, Tianjin 300308 (CN); GUO, Henghua, Hefei Anhui 231131 (CN); LIU, Pingping, Tianjin 300308 (CN); ZHANG, Dongzhu, Hefei Anhui 231131 (CN); TANG, Jinlei, Tianjin 300308 (CN); HAN, Chengxiu, Bayannaoer Inner Mongolia 015000 (CN); TANG, Siqing, Bayannaoer Inner Mongolia 015000 (CN); LIU, Shupeng, Hefei Anhui 231131 (CN); MA, Yanhe, Tianjin 300308 (CN)
(74) Representative: Biggi, Cristina
(86) International application number: PCT/CN2020/137780
(87) International publication number: WO 2021/227500

(57) **Abstract**

Related are a recombinant microorganism for producing L-valine, a construction method and an application thereof. Through transferring an acetohydroxy acid reductoisomerase gene and/or an amino acid dehydrogenase gene into a microorganism, and enhancing activity of an acetohydroxy acid reductoisomerase and/or an amino acid dehydrogenase, the titer and yield of L-valine generated by *Escherichia coli* may be improved, and L-valine was produced by one-step anaerobic fermentation.

## Description

### Technical Field

The disclosure relates to a construction method of a recombinant microorganism for producing L-valine, the recombinant microorganism obtained by the construction method, specifically recombinant *Escherichia coli,* and a method for producing L-valine through a fermentation method.

### Background

L-valine, as one of three branched-chain amino acids (BCAA), is an essential amino acid. It cannot be synthesized in humans and animals, and can only be acquired by supplementation in vitro. Nowadays, L-valine has been widely used in the fields of food and medicine, mainly including food additive, nutritional supplement and flavoring agent and the like; and widely used in preparation of cosmetics, as precursors of antibiotics or herbicides and the like. Additionally, along with the increasing demands for feed quality and ratio, the role of L-valine in the feed additive industry will become more and more important in the future. And this will result the expand of its demand and great potential in the future market.

L-valine can be directly synthesized by microbial cells. However, the production capacity of wild-type cells is greatly limited by the intracellular regulatory network, such as feedback inhibition and so on. To obtain a strain capable of efficiently producing L-valine by fermentation process, these self-regulatory mechanisms of the microbial cells must be effectively eliminated. At present,

L-valine is mainly produced through fermentation method in the world. Strains used for fermentative production of L-valine are mostly obtained by mutagenesis, and original strains mainly include Corynebacterium glutamicum, Brevibacterium flavum and the like. For example, starting from a Brevibacterium flavum, Ning Chen et al. produced mutant strains by using protoplast ultraviolet mutagenesis in combination with the DES chemical mutagenesis. Finally, an efficiently L-valine-producing strain TV2564 was screened and selected. But a notable fact is that strains obtained by traditional mutagenesis have many limites, such as strong randomness, unclear genetic background, producing by-products during fermentation, and not easy to modified for obtaining much more highly efficient strains.

With the rapid development of synthetic biology and metabolic engineering in recent years, recombinant engineered strains have been developed and achieved good results. These strains can efficiently produce L-valine, have clear genetic background, and are easy to cultivated. In 2007, Sang Yup Lee's group developed an L-valine-producing strain starting from the Escherichia coli W3110 by in combination of different strategies, such as the rational metabolic engineering, transcriptome analysis and genetic modification, and gene knockout. This strain can produce L-valine in batch culture under aerobic conditions. But it needs continuous oxygen supply during the culture process and L-isoleucine needs to be added to ensure the normal growth.

Xie xixian et al. integrate an encoding gene *alsS* of *Bacillus subtilis* acetolactate synthase to release the feedback inhibition of L-valine on a synthetic pathway, and integrate a mutant gene spoTM of *Escherichia coli* ppGpp3'-pyrophosphohydrolase to enhance supply of a pyruvate, and enhance a level of L-valine generated by shake flask fermentation of an original strain VHY03.

Most of the publicly reported L-valine fermentation production is achieved through aerobic or two-step fermentation at present. However, the aerobic process requires air during a production process and consumes a lot of energy; more critically, a considerable part of a carbon source enters a tricarboxylic acid cycle (TCA) and is consumed by cell growth resulting that the yield of L-valine is always much lower than the theoretical maximum. Compared with the aerobic process, the anaerobic process has advantages of low energy consumption and high yield of L-valine. The air is not needed during a production process, and the energy is greatly saved; and a yield of L-valine is usually close to the theoretical maximum. The anaerobic fermentation of amino acid production is firstly achieved in a process of alanine production. At present, there are no reports of pure anaerobic fermentation processes for production of other amino acids.

In addition, the overexpression of key genes involved in the metabolic engineering of L-valine strains are always plasmid-borne, so that antibiotics need to be added to maintain the existence of the plasmid during the fermentation process, a production cost is increased and a risk of plasmid loss exists in industrial production.

Therefore, there is still a need in the field to provide high-yield, energy-saving, simple and stable recombinant microorganisms for producing L-valine and a corresponding production and preparation method for L-valine.

### Summary

It is discovered in the disclosure that through enhancing activity of an acetohydroxy acid isoreductase and/or an amino acid dehydrogenase, titer and yield of L-valine generated by *Escherichia coli* can be improved, and one-step anaerobic fermentation of L-valine can be achieved.

The first aspect of the disclosure is to provide a construction method for construction of a recombinant microorganism producing L-valine. The recombinant microorganism obtained by this method has stable genetic background and balanced L-valine reducing power, and is suitable for one-step anaerobic fermentation.

In the present disclosure, "enhancement" of enzyme activity refers to enhancement of intracellular activity of one or more enzymes which has corresponding gene in the microogranism. The enhancement of the activity may be achieved by any suitable methods known in the field, for example, by overexpression, it includes but not limited to increasing the copy number of the gene or allele, modifying a nucleotide sequence for guiding or controlling gene expression, using a strong promoter to increase protein activity or concentration by 10%-500% compared to an initial microbial level.

In one embodiment, an acetohydroxy acid isoreductase gene or/and an amino acid dehydrogenase gene are transferred into a microorganism, so that the enzyme activity is enhanced.

In one embodiment, the acetohydroxy acid isoreductase gene and the amino acid dehydrogenase gene transferred in the disclosure are exogenous to the microorganism transferred. The acetohydroxy acid isoreductase gene and the amino acid dehydrogenase gene may be a corresponding gene from any microorganisms such as *Lactococcus,* and *Bacillus.*

In one embodiment, the acetohydroxy acid isoreductase gene and/or amino acid dehydrogenase gene are NADH-dependent.

Most of reducing power types of cell generation under anaerobic conditions is NADH. In order to achieve high-efficiency generation of L-valine under anaerobic conditions, the problem of cofactor imbalance must be solved. In one embodiment of the disclosure, the NADH-dependent acetohydroxy acid isoreductase gene and/or amino acid dehydrogenase gene may be selected to consume excessive NADH under anaerobic conditions and solve the problem of reducing power imbalance during the anaerobic fermentation.

In one embodiment, the acetohydroxy acid isoreductase gene is *ilvC* or *KARI;* and the amino acid dehydrogenase gene is a leucine dehydrogenase gene.

In one preferred embodiment, the acetohydroxy acid isoreductase gene is *KARI,* and the leucine dehydrogenase gene is *leuDH.*

The acetohydroxy acid isoreductase gene and the amino acid dehydrogenase gene may exist in the microorganism in any suitable forms known in the field, for example, in the form of a plasmid, or the form of being integrated into a genome. In one embodiment, the enzyme encoding gene integrated into the genome is placed under the control of a suitable regulatory element.

The regulatory element is selected from an M1-46 artificial regulatory element, an M1-93 artificial regulatory element or a RBS5 artificial regulatory element.

In one embodiment, the M1-46 artificial regulatory element regulates an *ilvC* gene.

In one embodiment, the M1-93 artificial regulatory element regulates a *leuDH* gene;

In one embodiment, the RBS5 artificial regulatory element regulates a *KARI* gene.

In one embodiment, the disclosure further includes the following modifications to one or more of the following enzyme genes of the above recombinant microorganism, so that the activity of these enzymes is reduced or inactivated.
(1) Knocking out a methylglyoxal synthase (*mgsA*) gene;
(2) knocking out a lactate dehydrogenase (*IdhA*) gene;
(3) knocking out phosphoacetyl transferase (pta) and/or acetate kinase (*ackA*) genes;
(4) knocking out propionate kinase (*tdcD*) and/or formate acetyltransferase (*tdcE*) genes;
(5) knocking out an alcohol dehydrogenase (*adhE*) gene; and
(6) knocking out fumarate reductase (*frd*) and/or pyruvate formate lyase (*pflB*) genes.

It may be understood by those skilled in the art that gene knockout may be performed in a manner known in the prior art, so that the activity of the enzyme is reduced or inactivated. The knockout operation is aimed at an endogenous enzyme gene of the original microorganism, so that the above endogenous enzyme activity of the microorganism is reduced or inactivated.

An encoding sequence of the enzyme gene in the above (1)-(6) may also be substituted with an encoding sequence of another gene by means of genetic engineering such as homologous recombination, thereby the above endogenous enzyme activity of the microorganism is reduced or inactivated. The gene to substitute these endogenous enzymes may be a gene to be enhanced for expression, such as the above *ilvC* gene, *KARI* gene or *leuDH* gene.

In one embodiment, it further includes enhancing activity of an acetolactate synthase (AHAS) and/or a dihydroxy acid dehydratase (*ilvD*) in the recombinant microorganism of the disclosure.

In one preferred embodiment, the AHAS is selected from *ilvBN, ilvGM* or *ilvIH,* and the activity of at least one of them is enhanced.

In one preferred embodiment, the activity of the AHAS is enhanced by releasing feedback inhibition of a valine to *ilvIH,* for example, the feedback inhibition of the valine to the *ilvIH* is released by mutating an *ilvH* gene.

For the involved acetolactate synthase used in biologically synthesizing L-valine, in addition to an isozyme II (herein also referred to as AHAS II), an isozyme III (herein also referred to as AHAS III) is also known. The AHASIII is encoded by an ilvIH operon, and the operon is formed by ilvl encoding a large subunit (catalytic subunit) and ilvH encoding a small subunit (control subunit). The AHASIII is feedback inhibited byL-valine. A reported method may be used to mutate the *ilvI* gene, such as amino acid substitution of ilvH 14 Gly→Asp (Vyazmensky, M. et al., "Biochemistry" 35:10339-10346 (1996)) and/or ilvH 17Ser→Phe (US6737255B2); and ilvH612 (De Felice et al., "Journal of Bacteriology" 120:1058-1067 (1974)) and the like.

In one embodiment, activity of a dihydroxy acid dehydratase (*ilvD*) in the recombinant microorganism of the disclosure is enhanced, for example, the *ilvD* gene is transferred into the microorganism to enhance the activity of the *ilvD.*

The activity of the AHAS and/or the dihydroxy acid dehydratase (*ilvD*) is enhanced, and it is operated optionally in combination with any one or more of the above modifications (1)-(6).

In one embodiment, the operation is performed in combination with the modification of the item (2).

In one embodiment, the operation is performed in combination with the modification of the item (6).

In one embodiment, the operation is performed in combination with the modifications of the item (2) and the item (5).

In one embodiment, the operation is performed in combination with the modifications of the items (1) and (3)-(6).

In one embodiment, the operation is performed in combination with the modifications of the items (1)-(6).

In one embodiment, optionally, the knockout of the item (1) is achieved by substituting the endogenous *mgsA* gene of the microorganism with the *ilvC* gene.

In one embodiment, the knockout of the item (6) is achieved by substituting the endogenous *pflB* gene of the microorganism with the *ilvD* gene, and/or substituting the endogenous *frd* gene of the microorganism with the *leuDH* gene.

In one embodiment, the knockout of the item (5) is achieved by substituting the endogenous *adhE* gene of the microorganism with the *KARI* gene.

The substitution may be that, in a mode known to those skilled in the art, an encoding sequence of a gene to be inserted is integrated into a substituted gene in the microbial chromosome, so that the gene encoding sequence in the original site is substituted with the encoding sequence of the integrated inserted gene.

Preferably, the substitution of the *KARI, ilvC, ilvD* and *leuDH* occur simultaneously, herein the *ilvC* gene is optionally knocked out again.

In one embodiment, the microorganism is *Escherichia coli.*

In one embodiment, the microorganism is *Escherichia coli* ATCC 8739.

In one embodiment, at least one regulatory element is used to regulate the genes encoding the above enzymes involved.

In one embodiment, the regulatory element is selected from an M1-46 artificial regulatory element, an M1-93 artificial regulatory element or a RBS5 artificial regulatory element.

In one embodiment, the M1-46 artificial regulatory element regulates an *ilvC* gene.

In one embodiment, the M1-93 artificial regulatory element regulates the *ilvD, leuDH, ilvBN* and *ilvGM* genes.

In one embodiment, the RBS5 artificial regulatory element regulates a *KARI gene.*

The regulatory element may be inserted at an upstream of the *ilvC* gene by a known genetic engineering method. The method includes, but is not limited to, inserting the sequence of the regulatory element into the upstream of the gene encoding sequence of the target enzyme by means of gene recombination, for example, by means of homologous recombination, so as to enhance the intensity of the target gene expression.

In one embodiment, herein the enzyme encoding gene and the regulatory element are integrated into the genome of the microorganism.

In one embodiment, a plasmid containing the enzyme encoding gene and the regulatory element sequence is transferred into the microorganism.

In one embodiment, the transfer, mutation or knockout of the target enzyme gene is completed by a method of integrating into the genome of the microorganism.

In one embodiment, the transfer, mutation or knockout of the enzyme gene is completed by a method of homologous recombination.

In one embodiment, the transfer, mutation or knockout of the enzyme gene is completed by a method of two-step homologous recombination.

The homologous recombination may be implemented by the homologous recombination system known to the related art, such as an *Escherichia coli* RecA recombination system or a Red recombination system, to transfer, mutate or knock out the target gene.

The two-step homologous recombination method to transfer, mutate or knock out the target gene includes the following steps (*Escherichia coli are* taken as an example):
(1) Preparation of a DNA fragment I: a pXZ-CS plasmid (Tan, et al., Appl Environ Microbiol, 2013, 79:4838-4844) DNA is used as a template, an amplification primer 1 is used to amplify the DNA fragment I, and used for the first step of homologous recombination;
(2) the first step of the homologous recombination: a pKD46 plasmid (Datsenko and Wanner 2000, Proc Natl Acad Sci USA 97:6640-6645) is transformed into *Escherichia coli,* and then the DNA fragment I is transformed into the *Escherichia coli* containing pKD46, a detection primer 1 is used to verify the transformed bacteria and select the correct colony;
(3) preparation of a DNA fragment II: the original *Escherichia coli* are used as a template, and an amplification primer 2 is used to amplify the DNA fragment II. The DNA fragment II is used for a second step of homologous recombination; and
(4) the second step of the homologous recombination: the DNA fragment II is transformed into the strain obtained in the second step; and a detection primer 2 is used to verify the transformed bacteria and select a correct colony.

The second aspect of the disclosure provides a recombinant microorganism for producing L-valine obtained by using the above construction method, specifically recombinant *Escherichia coli,* it contains an acetohydroxy acid isoreductase and/or an amino acid dehydrogenase gene,

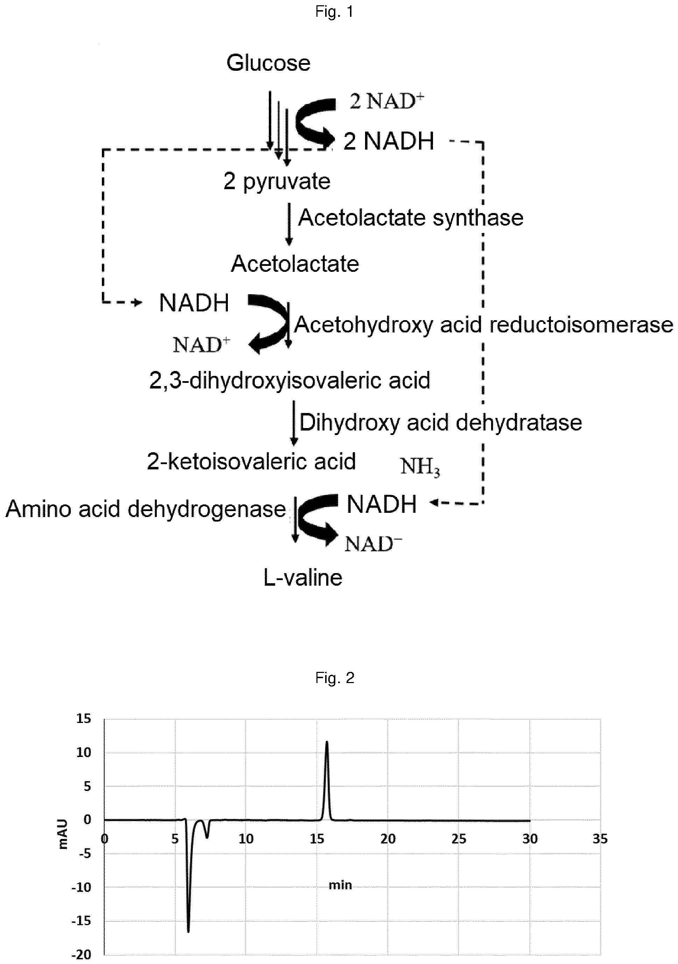

In one embodiment, the *Escherichia coli ATCC* 8739 is used as an original strain, and coupling of intracellular cofactor NADH supply and cell growth is achieved through the gene homologous recombination, thereby coupling (Fig. 1) of the cell growth and L-valine production under anaerobic conditions is achieved.

In one embodiment, the recombinant *Escherichia coli* obtained by the above construction method undergo metabolic evolution, for example, through 50 generations, 70 generations, 80 generations, 90 generations, 100 generations, and 120 generations, the recombinant *Escherichia coli for* highly producing L-valine are obtained. In one embodiment, a recombinant *Escherichia coli* strain producing L-valine is obtained after 105 generations of metabolic evolution. It is preserved in China General Microbiological Culture Collection Center (CGMCC) on March 6, 2020, the Depository Authority is located at No. 3, Courtyard 1, Beichen West Road, Chaoyang District, Beijing, the deposit number is: CGMCC 19458, and the classification name is *Escherichia coli.*

The third aspect of the disclosure is an application of the recombinant microorganism obtained by the above method in producing L-valine.

The fourth aspect of the disclosure is a method for fermentatively producing L-valine using the recombinant microorganism obtained by the above construction method, including: (1) fermenting the recombinant microorganism obtained by the above construction method; and (2) separating and harvesting L-valine.

In one embodiment, the fermentation is anaerobic fermentation.

In one embodiment, the anaerobic fermentation includes the following steps:
(1) seed culture: a clone on a plate is picked and inoculated into a seed culture medium at 37°C, and shake culture is performed, to obtain seed culture solution; and
(2) fermentation culture: the seed culture solution is inoculated into fermentation culture medium, and culturing at 37°C and 150 rpm for 4 days, to obtain fermentation solution. The pH during fermentation process is controlled at 7.0. No air was sparged during the fermentation.

Herein the seed culture medium is formed by the following components (a solvent is water): Glucose 20 g/L, corn syrup dry powder 10 g/L, KH₂PO₄ 8.8 g/L, (NH₄)₂SO₄ 2.5 g/L, and MgSO₄ •7H₂O 2 g/L.

The fermentation culture medium and the seed culture medium have the same components, and a difference is only that the glucose concentration is 50 g/L.

The beneficial effects of the disclosure:
(1) Compared with previous production methods and strains, the disclosure achieves the one-step anaerobic fermentation production of L-valine, reduces the production cost and improves the yield of L-valine.
(2) The disclosure preferably constructs a genetically stable L-valine production strain by modified directly on the genome of the recombinant microorganism rather than in the form of plasmid, and additional addition of substances such as antibiotics and inducers does not require, and the production process is stable and easy to operate.
(3) Through metabolic evolution, the titer and yield of L-valine and cell tolerance are improved significantly in the recombinant microorganisms.

### Preservation of Biological Material

Recombinant *Escherichia coli* Sval065 constructed in the disclosure are classified and named: *Escherichia coli.* A biological material is submitted for preservation on March 6, 2020, Depository Authority: China General Microbiological Culture Collection Center (CGMCC), the preservation address is No. 3, Courtyard 1, Beichen West Road, Chaoyang District, Beijing, Telephone: 010-64807355, and the deposit number is CGMCC No. 19458.

### Brief Description of the Drawings

Drawings of the description for constituting a part of the present disclosure are used to provide further understanding of the disclosure. Exemplary embodiments of the disclosure and descriptions thereof are used to explain the disclosure, and do not constitute improper limitation to the disclosure. In the drawings:
Fig. 1: L-valine synthesis pathway.
Fig. 2: Determination of a standard substance of L-valine measured by high performance liquid chromatography.
Fig. 3: Determination of fermentation solution components of strain Sval064 by high performance liquid chromatography.
Fig. 4: Construction of the strain Sval065 by metabolic evolution.
Fig. 5: Determination of a standard substance of L-valine by high performance liquid chromatography.
Fig. 6: Determination of fermentation solution components of strain Sval065 by high performance liquid chromatography.

### Detailed Description of the Embodiments

The disclosure is further described by the following embodiments, but any embodiments or combinations thereof should not be interpreted as limiting a scope or an embodiment of the disclosure. The scope of the disclosure is defined by appended claims. In combination with the description and common knowledge in the field, those of ordinary skill in the art may clearly understand the scope defined by the claims. Without departing from the spirit and scope of the disclosure, those skilled in the art may make any modifications or changes to technical schemes of the disclosure, and such modifications and changes are also included in the scope of the disclosure.

Experimental methods used in the following embodiments are conventional methods unless otherwise specified. Materials, reagents and the like used in the following embodiments may be obtained from commercial sources unless otherwise specified.

Strains and plasmids constructed in this research are shown in Table 1, and primers used are shown in Table 2.

**Table 1: Strains and plasmids used in the disclosure**

| **Strain** | **Related characteristics** | **Sources** |
|---|---|---|
| ATCC 8739 | Wild type | Laboratory preservation |
| M1-93 | ATCC 8739, FRT-Km-FRT::M1-93::lacZ | Lu, et al., Appl Microbiol Biotechnol, 2012,93:2455-2462 |
| M1-46 | ATCC 8739, FRT-Km-FRT::M1-46::lacZ | Lu, et al., Appl Microbiol Biotechnol, 2012,93:2455-2462 |
| Sval001 | ATCC 8739, mgsA::cat-sacB | Constructed by the disclosure |
| Sval002 | Sval001, ΔmgsA | Constructed by the disclosure |
| Sval003 | Sval002, ldhA::cat-sacB | Constructed by the disclosure |
| Sval004 | Sval003, ΔldhA | Constructed by the disclosure |
| Sval005 | Sval004, ackA-pta::cat-sacB | Constructed by the disclosure |
| Sval006 | Sval005, Δ ackA-pta | Constructed by the disclosure |
| Sval007 | Sval006, tdcDE::cat-sacB | Constructed by the disclosure |
| Sval008 | Sval007, ΔtdcDE | Constructed by the disclosure |
| Sval009 | Sval008, adhE::cat-sacB | Constructed by the disclosure |
| Sval010 | Sval009, ΔadhE | Constructed by the disclosure |
| Sval011 | Sval010, mgsA::cat-sacB | Constructed by the disclosure |
| Sval012 | Sval011, mgsA::ilvC | Constructed by the disclosure |
| Sval013 | Sval012, mgsA::cat-sacB::ilvC | Constructed by the disclosure |
| Sval014 | Sval013, mgsA::M1-46-ilvC | Constructed by the disclosure |
| Sval015 | Sval014, pflB::cat-sacB | Constructed by the disclosure |
| Sval016 | Sval015, pflB::ilvD | Constructed by the disclosure |
| Sval017 | Sval016, pflB::cat-sacB::ilvD | Constructed by the disclosure |
| Sval018 | Sval017, pflB::RBS4-ilvD | Constructed by the disclosure |
| Sval019 | Sval018, cat-sacB::ilvB | Constructed by the disclosure |
| Sval020 | Sval019, M1-93:: ilvB | Constructed by the disclosure |
| Sval021 | Sval020, cat-sacB::ilvG | Constructed by the disclosure |
| Sval022 | Sval021, M1-93:: ilvG | Constructed by the disclosure |
| Sval023 | Sval022, ilvH::cat-sacB | Constructed by the disclosure |
| Sval024 | Sval023, ilvH:: ilvH* | Constructed by the disclosure |
| Sval025 | Sval024, frd::cat-sacB | Constructed by the disclosure |
| Sval026 | Sval025, frd::M1-93-leuDH | Constructed by the disclosure |
| Sval061 | Sval026, adhE::cat-sacB | Constructed by the disclosure |
| Sval062 | Sval061, adhE::RBS5-kari | Constructed by the disclosure |
| Sval063 | Sval062, mgsA::cat-sacB | Constructed by the disclosure |
| Sval064 | Sval063, ΔmgsA | Constructed by the disclosure |
| Sval065 | metabolic evolution of Sval064 for 105 generations | Constructed by the disclosure |
| **Plasmid** | **Related characteristics** | **Sources** |
| pUC57-M1 -93-leuDH | Artificial regulatory element M1-93 and chemically synthesized gene *leuDH* are linked to a pUC57 vector together | Nanjing Genscript Biotechnology Co., Ltd. |
| pUC57-RBS5-kari | Artificial regulatory element RBS5 and chemically synthesized gene kari are linked to the pUC57 vector together | Nanjing Genscript Biotechnology Co., Ltd. |

**Table 2: Primers used in the disclosure**

| **Primer name** | **Sequence** | **Sequen ce number** |
|---|---|---|
| mgsA-cs-up | gtaggaaagttaactacggatgtacattatggaactgacgactcgcactt TGTGACGGAAGATCACTTCGCAG | 1 |
| mgsA-cs-down | gcgtttgccacctgtgcaatattacttcagacggtccgcgagataacgctT TATTTGTTAACTGTTAATTGTCCT | 2 |
| XZ-mgsA-up | cagctcatcaaccaggtcaa | 3 |
| XZ-mgsA-dow n | aaaagccgtcacgttattgg | 4 |
| mgsA-del-dow n | gcgtttgccacctgtgcaatattacttcagacggtccgcgagataacgcta agtgcgagtcgtcagttcc | 5 |
| mgsA-ilvC-up | gtaggaaagttaactacggatgtacattatggaactgacgactcgcactt ATGGCTAACTACTTCAATACac | 6 |
| mgsA-ilvC-do wn | gcgtttgccacctgtgcaatattacttcagacggtccgcgagataacgctT TAACCCGCAACAGCAATACGtttc | 7 |
| mgsA-Pcs-up | gtaggaaagttaactacggatgtacattatggaactgacgactcgcactt TGTGACGGAAGATCACTTCGCAG | 8 |
| mgsA-Pcs-do | agctgtgccagctgctggcgcagattcagtGTATTGAAGTAGTTA | 9 |
| wn | GCCATTTATTTGTTAACTGTTAATTGTCCT | |
| mgsA-P46-up | gtaggaaagttaactacggatgtacattatggaactgacgactcgcactt TTATCTCTGGCGGTGTTGAC | 10 |
| ilvC-P46-down | agctgtgccagctgctggcgcagattcagtGTATTGAAGTAGTTA GCCATAGCTGTTTCCTGGTTTAAACCG | 11 |
| ilvC-YZ347-do wn | cgcactacatcagagtgctg | 12 |
| ldhA-cs-up | ttcaacatcactggagaaagtcttatgaaactcgccgtttatagcacaaa TGTGACGGAAGATCACTTCGCAG | 13 |
| ldhA-cs-down | agcggcaagattaaaccagttcgttcgggcaggtttcgcctttttccagaT TATTTGTTAACTGTTAATTGTCCT | 14 |
| XZ-ldhA-up | GATAACGGAGATCGGGAATG | 15 |
| XZ- ldhA-down | CTTTGGCTGTCAGTTCACCA | 16 |
| ldhA-del-down | agcggcaagattaaaccagttcgttcgggcaggtttcgcctttttccagattt gtgctataaacggcgagt | 17 |
| ackA-cs-up | aggtacttccatgtcgagtaagttagtactggttctgaactgcggtagttTG TGACGGAAGATCACTTCGCAG | 18 |
| pta-cs-down | ggtcggcagaacgctgtaccgctttgtaggtggtgttaccggtgttcagaT TATTTGTTAACTGTTAATTGTCCT | 19 |
| XZ-ackA-up | cgggacaacgttcaaaacat | 20 |
| XZ-pta-down | attgcccatcttcttgttgg | 21 |
| ackA-del-down | ggtcggcagaacgctgtaccgctttgtaggtggtgttaccggtgttcagaa actaccgcagttcagaacca | 22 |
| tdcDE-cs-up | ccgtgattggtctgctgaccatcctgaacatcgtatacaaactgttttaaTG TGACGGAAGATCACTTCGCAG | 23 |
| tdcDE-cs-dow n | cgcctggggcacgttgcgtttcgataatctttttcatacatcctccggcgTT ATTTGTTAACTGTTAATTGTCCT | 24 |
| XZ-tdcDE-up | TGATGAGCTACCTGGTATGGC | 25 |
| XZ-tdcDE-dow n | CGCCGACAGAGTAATAGGTTTTAC | 26 |
| tdcDE-del-dow n | cgcctggggcacgttgcgtttcgataatctttttcatacatcctccggcgttaa aacagtttgtatacgatgttcag | 27 |
| adhE-cs-up | ATAACTCTAATGTTTAAACTCTTTTAGTAAATCACAG TGAGTGTGAGCGCTGTGACGGAAGATCACTTCGC A | 28 |
| adh E-cs-down | CCGTTTATGTTGCCAGACAGCGCTACTGATTAAGC GGATTTTTTCGCTTTTTATTTGTTAACTGTTAATTGT CCT | 29 |
| adh E-del-dow n | CCGTTTATGTTGCCAGACAGCGCTACTGATTAAGC GGATTTTTTCGCTTTGCGCTCACACTCACTGTGAT TTAC | 30 |
| XZ-adhE-up | CATGCTAATGTAGCCACCAAA | 31 |
| XZ-adhE-down | TTGCACCACCATCCAGATAA | 32 |
| adhE-RBS5-u p | ATAACTCTAATGTTTAAACTCTTTTAGTAAATCACAG TGAGTGTGAGCGCTTATCTCTGGCGGTGTTGAC | 33 |
| adh E-kari-dow n | CCGTTTATGTTGCCAGACAGCGCTACTGATTAAGC GGATTTTTTCGCTTTTTAGATAACTTTTTTCTTCA | 34 |
| pflB-CS-up | aaacgaccaccattaatggttgtcgaagtacgcagtaaataaaaaatcc aTGTGACGGAAGATCACTTCGCAG | 35 |
| pflB-CS-down | CGGTCCGAACGGCGCGCCAGCACGACGACCGTC TGGGGTGTTACCCGTTTTTATTTGTTAACTGTTAAT TGTCCT | 36 |
| pflB-ilvD-up | aaacgaccaccattaatggttgtcgaagtacgcagtaaataaaaaatcc aatgcctaagtaccgttccgc | 37 |
| pflB-ilvD-down | CGGTCCGAACGGCGCGCCAGCACGACGACCGTC | 38 |
| | TGGGGTGTTACCCGTTTttaacccccagtttcgatttatc | |
| XZ-pflB-up600 | CTGCGGAGCCGATCTCTTTAC | 39 |
| XZ-pflB-down | CGAGTAATAACGTCCTGCTGCT | 40 |
| pflB-Pcs-up | aaacgaccaccattaatggttgtcgaagtacgcagtaaataaaaaatcc aTGTGACGGAAGATCACTTCGCA | 41 |
| pflB-Pcs-down | CCCGCCATATTACGACCATGAGTGGTGGTGGCGG AACGGTACTTAGGCATTTATTTGTTAACTGTTAATT GTCCT | 42 |
| pflB-Pro-up | AAACGACCACCATTAATGGTTGTCGAAGTACGCAG TAAATAAAAAATCCATTATCTCTGGCGGTGTTGAC | 43 |
| ilvD-Pro-down | cccgccatattacgaccatgagtggtggtggcggaacggtacttaggcat TGCTGACCTCCTGGTTTAAACGTACATG | 44 |
| ilvD-YZ496-do wn | caaccagatcgagcttgatg | 45 |
| XZ-frd-up | TGCAGAAAACCATCGACAAG | 46 |
| Xz-frd-down | CACCAATCAGCGTGACAACT | 47 |
| frd-cs-up | GAAGGCGAATGGCTGAGATGAAAAACCTGAAAATT GAGGTGGTGCGCTATTGTGACGGAAGATCACTTC GCA | 48 |
| frd-cs-down | TCTCAGGCTCCTTACCAGTACAGGGCAACAAACA GGATTACGATGGTGGCTTATTTGTTAACTGTTAATT GTCCT | 49 |
| frd-M93-up | GAAGGCGAATGGCTGAGATGAAAAACCTGAAAATT GAGGTGGTGCGCTATTTATCTCTGGCGGTGTTGAC | 50 |
| frd-leuDH-dow n | TCTCAGGCTCCTTACCAGTACAGGGCAACAAACA GGATTACGATGGTGGCTTAACGGCCGTTCAAAATA TTTTTTTC | 51 |
| ilvB pro-catup | ctgacgaaacctcgctccggcggggtttttgttatctgcaattcagtacTG | 52 |
| | TGACGGAAGATCACTTCGCA | |
| ilvB pro-catdown | tctgcgccggtaaagcgcttacgcgtcgatgttgtgcccgaacttgccatT TATTTGTTAACTGTTAATTGTCCT | 53 |
| ilvB pro-up | ctgacgaaacctcgctccggcggggtttttgttatctgcaattcagtacTT ATCTCTGGCGGTGTTGAC | 54 |
| ilvB pro-down | tctgcgccggtaaagcgcttacgcgtcgatgttgtgcccgaacttgccatA GCTGTTTCCTGGTTTAAAC | 55 |
| ilvB pro-YZup | gttctgcgcggaacacgtatac | 56 |
| ilvB pro- YZdown | ccgctacaggccatacagac | 57 |
| ilvG pro-catup | tgaactaagaggaagggaacaacattcagaccgaaattgaatttttttca TGTGACGGAAGATCACTTCGCA | 58 |
| ilvG pro-catdown | ttcacaccctgtgcccgcaacgcatgtaccacccactgtgcgccattcatT TATTTGTTAACTGTTAATTGTCCT | 59 |
| ilvG pro-up | tgaactaagaggaagggaacaacattcagaccgaaattgaatttttttca TTATCTCTGGCGGTGTTGAC | 60 |
| ilvG pro-down | ttcacaccctgtgcccgcaacgcatgtaccacccactgtgcgccattcat AGCTGTTTCCTGGTTTAAACG | 61 |
| ilvG pro-YZup | gcataagatatcgctgctgtag | 62 |
| ilvG p-YZdown | gccagttttgccagtagcac | 63 |
| ilvH*-cat-up | agaacctgattatgCGCCGGATATTATCAGTCTTACTCGA AAATGAATCATGTGACGGAAGATCACTTCGCA | 64 |
| ilvH* -cat-down | TTCATCGCCCACGGTCTGGATGGTCATACGCGATA ATGTCGGATCGTCGGTTATTTGTTAACTGTTAATTG TCCT | 65 |
| ilvH*-mut-up | agaacctgattatgCGCCGGATATTATCAGTCTTACTCGA AAATGAATCAGaCGCGTTATtCCGCGTGATTGGC | 66 |
| ilvH* -mut-dow n | CACACCAGAGCGAGCAACCTC | 67 |
| ilvH* -mutYZ-u p | atgagctg gaaagcaaacttagc | 68 |

### Example 1: Knockout of methylglyoxal synthase encoding gene mgsA in ATCC 8739 strain

Started from *Escherichia coli ATCC* 8739, a two-step homologous recombination method is used to knock out the methylglyoxal synthase encoding gene *mgsA,* and specific steps are as follows.

In a first step, a pXZ-CS plasmid DNA is used as a template, 2719 bp of a DNA fragment I is amplified by using primers mgsA-cs-up/mgsA-cs-down, and used for the first step of homologous recombination.

An amplification system is: Phusion 5X buffer (NewEngland Biolabs) 10 µl, dNTP (10 mM for each dNTP) 1 µl, DNA template 20 ng, primers (10 µM) 2 µl each, Phusion High-Fidelity DNA polymerase (2.5 U/µl) 0.5 µl, distilled water 33.5 µl, and a total volume is 50 µl.

Amplification conditions are 98°C pre-denaturation for 2 minutes (1 cycle); 98°C denaturation for 10 seconds, 56°C annealing for 10 seconds, 72°C extension for 2 minutes (30 cycles); and 72°C extension for 10 minutes (1 cycle).

The above DNA fragment I is used for the first homologous recombination: firstly, a pKD46 plasmid (purchased from the Coil Genetic Stock Center (CGSC) of Yale University, CGSC#7739) is transformed into *Escherichia coli* ATCC 8739 by an electrotransformation method, and then the DNA fragment I is electrotransformed to the *Escherichia coli ATCC* 8739 with the pKD46.

Electrotransformation conditions are as follows: firstly, electrotransformation competent cells of the *Escherichia coli ATCC* 8739 with the pKD46 plasmid are prepared; 50 µl of the competent cells are placed on ice, and 50 ng of the DNA fragment I is added. The mixture was placed on ice for 2 minutes, and transferred into a 0.2 cm MicroPulser Electroporation Cuvette (Bio-Rad). The electroporation was carried with the MicroPulser (Bio-Rad) electroporation apparatus and the electric voltage was 2.5 kV. After electric shock, 1 ml of LB medium was quickly added into the electroporation cuvette, and transferred into a test tube after pipetting five times. The culture was incubated at 30°C with shaking at 75 rpm for 2 hours. 200 µl of culture was spread onto a LB plate containing ampicillin (a final concentration is 100 µg/ml) and chloramphenicol (a final concentration is 34 µg/ml). After being cultured overnight at 30°C, colonies were verified with primers XZ-mgsA-up/XZ-mgsA-down, and a correct colony amplification product is a 3646 bp fragment. A correct single colony was selected, and named as Sval001.

In a second step, a genomic DNA of wild-type *Escherichia coli* ATCC 8739 is used as template, and 566 bp of a DNA fragment II is amplified with primers XZ-mgsA-up/mgsA-del-down. DNA fragment II is used for the second homologous recombination. Amplification conditions and system are the same as those described in the first step. The DNA fragment II is electrotransformed into strain Sval001.

Electrotransformation conditions are as follows: firstly, electrotransformation competent cells of the Sval001 with the pKD46 plasmid were prepared; 50 µl of the competent cells were placed on ice, and 50 ng of a DNA fragment II is added. The mixture was placed on ice for 2 minutes, and transferred into a 0.2 MicroPulser Electroporation Cuvette (Bio-Rad). The electroporation was carried with the MicroPulser (Bio-Rad) electroporation apparatus and the electric voltage was 2.5 kV. After electric shock, 1 ml of LB medium was quickly added into the electroporation cuvette, and transferred into a test tube after pipetting five times. The culture was incubated at 30°C with shaking at 75 rpm for 4 hours. The culture was then transferred into LB medium containing 10% sucrose but without a sodium chloride (50 ml of a medium is loaded in 250 ml of a flask), and after being cultured for 24 hours, it is streak-cultured on an LB solid medium containing 6% sucrose without a sodium chloride. The correct clone was verified by colony PCR amplification with primers XZ-mgsA-up/mgsA-del-down, and a correct colony amplification product was 1027 bp. A correct single colony is then selected, and named as Sval002.

### Example 2: Knockout of lactate dehydrogenase encoding gene IdhA

Started from Sval002, and a lactate dehydrogenase encoding gene *IdhA* is knocked out by a two-step homologous recombination method. Specific steps are as follows:
In a first step, a pXZ-CS plasmid DNA is used as a template, 2719 bp of a DNA fragment I is amplified by using primers ldhA-cs-up/ldhA-cs-down, and used for the first step of the homologous recombination. Amplification system and amplification conditions are the same as those described in Example 1. The DNA fragment I is electrotransformed to the Sval002.

The DNA fragment I is used for the first homologous recombination: firstly, a pKD46 plasmid is transformed into *Escherichia coli* Sval002 by an electrotransformation method, and then the DNA fragment I is electrotransformed into the *Escherichia coli* Sval002 with the pKD46.

Electrotransformation conditions and steps are the same as the first step method for the *mgsA* gene knockout described in Example 1. 200 µl of culture solution is spreaded onto a LB plate containing ampicillin (a final concentration is 100 µg/ml) and chloramphenicol (a final concentration is 34 µg/ml). After being cultured overnight at 30°C, colonies were PCR verified with primers XZ-IdhA-up/XZ-IdhA-down, and a correct PCR product should be 3448 bp. A correct single colony is picked, and named as Sval003.

In a second step, a DNA of wild-type *Escherichia coli ATCC* 8739 is used as a template, and 476 bp of a DNA fragment II is amplified with primers XZ-ldhA-up/ldhA-del-down. The DNA fragment II is used for the second homologous recombination. The DNA fragment II is electrotransformed into strain Sval003.

Electrotransformation conditions and steps are the same as the second step method for the *mgsA* gene knockout described in Example 1. Colony PCR is used to verify clones using primers XZ-IdhA-up/XZ-IdhA-down, and a correct colony amplification product is 829 bp . A correct single colony is picked, and named as Sval004.

### Example 3: Knockout of phosphoacetyl transferase encoding gene pta and acetate kinase encoding gene ackA

Started from Sval004, a two-step homologous recombination method is used to knock out a phosphoacetyl transferase encoding gene *pta* and an acetate kinase encoding gene *ackA.* Specific steps are as follows.

In a first step, a pXZ-CS plasmid DNA is used as a template, 2719 bp of a DNA fragment I is amplified by using primers ackA-cs-up/pta-cs-down, and used for the first step of the homologous recombination. Amplification system and amplification conditions are the same as those described in Example 1. The DNA fragment I is electrotransformed to the Sval004.

The DNA fragment I is used for the first homologous recombination: firstly, a pKD46 plasmid is transformed into *Escherichia coli* Sval004 by an electrotransformation method, and then the DNA fragment I is electrotransformed into the *Escherichia coli* Sval004 with the pKD46.

Electrotransformation conditions and steps are the same as the first step method for the *mgsA* gene knockout described in Example 1. 200 µl of culture solution is spreaded onto a LB plate containing ampicillin (a final concentration is 100 µg/ml) and chloramphenicol (a final concentration is 34 µg/ml). After being cultured overnight at 30°C, colonies were PCR verified with primers XZ-ackA-up/XZ-pta-down, and a correct PCR product should be 3351 bp. A correct single colony is picked, and named as Sval005.

In a second step, a DNA of wild-type *Escherichia coli ATCC* 8739 is used as a template, and 371 bp of a DNA fragment II is amplified with primers XZ-ackA-up/ackA-del-down. The DNA fragment II is used for the second homologous recombination. The DNA fragment II is electrotransformed into strain Sval005.

Electrotransformation conditions and steps are the same as the second step method for the *mgsA* gene knockout described in Example 1. Colony PCR is used to verify clones using primers XZ-ackA-up/XZ-pta-down, and a correct colony amplification product is 732 bp . A correct single colony is picked, and named as Sval006.

### Example 4: Knockout of propionate kinase encoding gene tdcD and formate acetyltransferase encoding gene tdcE

Started from Sval006, a two-step homologous recombination method is used to knock out the propionate kinase encoding gene *tdcD* and the formate acetyltransferase encoding gene *tdcE.* Specific steps are as follows.

In a first step, a pXZ-CS plasmid DNA is used as a template, 2719 bp of a DNA fragment I is amplified by using primers tdcDE-cs-up/tdcDE-cs-down, and used for the first step of the homologous recombination. Amplification system and amplification conditions are the same as those described in Example 1. The DNA fragment I is electrotransformed to the Sval006.

The DNA fragment I is used for the first homologous recombination: firstly, a pKD46 plasmid is transformed into *Escherichia coli* Sval006 by an electrotransformation method, and then the DNA fragment I is electrotransformed into the *Escherichia coli* Sval006 with the pKD46.

Electrotransformation conditions and steps are the same as the first step method for the *mgsA* gene knockout described in Example 1. 200 µl of culture solution is spreaded onto a LB plate containing ampicillin (a final concentration is 100 µg/ml) and chloramphenicol (a final concentration is 34 µg/ml). After being cultured overnight at 30°C, colonies were PCR verified with primers XZ-tdcDE-up/ XZ-tdcDE-down, and a correct PCR product should be 4380 bp. A correct single colony is picked, and named as Sval007.

In a second step, a DNA of wild-type *Escherichia coli ATCC* 8739 is used as a template, and 895 bp of a DNA fragment II is amplified with primers XZ-tdcDE-up/tdcDE-del-down. The DNA fragment II is used for the second homologous recombination. The DNA fragment II is electrotransformed into strain Sval007.

Electrotransformation conditions and steps are the same as the second step method for the *mgsA* gene knockout described in Example 1. Colony PCR is used to verify clones using primers XZ-tdcDE-up/ XZ-tdcDE-down, and a correct colony amplification product is 1761 bp . A correct single colony is picked, and named as Sval008.

### Example 5: Knockout of alcohol dehydrogenase gene adhE

Started from Sval008, a two-step homologous recombination method is used to knock out the alcohol dehydrogenase gene *adhE.* Specific steps are as follows.

In a first step, a pXZ-CS plasmid DNA is used as a template, 2719 bp of a DNA fragment I is amplified by using primers adhE-cs-up /adhE-cs-down, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval008 by an electrotransformation method, and then the DNA fragment I is electrotransformed into the *Escherichia coli* Sval008 with the pKD46.

Electrotransformation conditions and steps are the same as the first step method for the *mgsA* gene knockout described in Example 1. 200 µl of culture solution is spreaded onto a LB plate containing ampicillin (a final concentration is 100 µg/ml) and chloramphenicol (a final concentration is 34 µg/ml). After being cultured overnight at 30°C, colonies were PCR verified with primers XZ-adhE-up/XZ-adhE-down, and a correct PCR product should be 3167 bp. A correct single colony is picked, and named as Sval009.

In a second step, a DNA of wild-type *Escherichia coli ATCC* 8739 is used as a template, and 271 bp of a DNA fragment II is amplified with primers XZ-adhE-up/XZ-adhE-down. The DNA fragment II is used for the second homologous recombination. The DNA fragment II is electrotransformed into strain Sval009.

Electrotransformation conditions and steps are the same as the second step method for the *mgsA* gene knockout described in Example 1. Colony PCR is used to verify clones using primers XZ-adhE-up/XZ-adhE-down, and a correct colony amplification product is 548 bp . A correct single colony is picked, and named as Sval010.

### Example 6: Integration of acetohydroxy acid reductoisomerase encoding gene ilvC in methylglyoxal synthase encoding gene mgsA site

Started from Sval010, an acetohydroxy acid reductoisomerase encoding gene *ilvC* from *Escherichia coli* is integrated into the methylglyoxal synthase encoding gene *mgsA* site through a two-step homologous recombination method. Specific steps are as follows.

In a first step, a cat-sacB fragment is integrated into the *mgsA* site of strain Sval010. PCR, integration, and verification of the cat-sacB fragment are exactly the same as the first step of the *mgsA* gene knockout in Example 1, and an obtained clone is named as Sval011.

In a second step, a DNA of wild-type *Escherichia coli ATCC* 8739 is used as a template, 1576 bp of a DNA fragment II is amplified by using primers mgsA-ilvC-up/mgsA-ilvC-down. The DNA fragment II is used for the second homologous recombination. The DNA fragment II is electrotransformed into strain Sval011.

Electrotransformation conditions and steps are the same as the second step method for the *mgsA* gene knockout described in Example 1. Colonies were PCR verified using primers XZ-mgsA-up/XZ-mgsA-down and sequenced, and a correct colony amplification product is 2503 bp . A correct single colony is picked, and named as Sval012.

### Example 7: Regulation of acetohydroxy acid reductoisomerase encoding gene ilvC

Started from Sval012, and an artificial regulatory element is used to regulate expression of the acetohydroxy acid reductoisomerase encoding gene *ilvC* integrated in methylglyoxal synthase encoding gene *mgsA* site. Specific steps are as follows.

In a first step, a pXZ-CS plasmid DNA is used as a template, 2719 bp of a DNA fragment I is amplified by using primers mgsA-Pcs-up/mgsA-Pcs-down, and used for the first step of the homologous recombination. Amplification system and amplification conditions are the same as those described in Example 1. The DNA fragment I is electrotransformed into the Sval012.

The DNA fragment I is used for the first homologous recombination: firstly, a pKD46 plasmid is transformed into *Escherichia coli* Sval012 by an electrotransformation method, and then the DNA fragment I is electrotransformed into the *Escherichia coli* Sval012 with the pKD46.

Electrotransformation conditions and steps are the same as the first step method for the *mgsA* gene knockout described in Example 1. 200 µl of culture solution is spreaded onto a LB plate containing ampicillin (a final concentration is 100 µg/ml) and chloramphenicol (a final concentration is 34 µg/ml). After being cultured overnight at 30°C, colonies were PCR verified with primers XZ-mgsA-up/ilvC-YZ347-down, and a correct PCR product should be 3482 bp. A correct single colony is picked, and named as Sval013.

In a second step, a genomic DNA of M1-46 (Lu, et al., Appl Microbiol Biotechnol, 2012,93:2455-2462) is used as a template, and 188 bp of a DNA fragment II is amplified by using primers mgsA-P46-up/ilvC-P46-down. The DNA fragment II is used for the second homologous recombination. The DNA fragment II is electrotransformed into strain Sval013.

Electrotransformation conditions and steps are the same as the second step method for the *mgsA* gene knockout described in Example 1. Colony PCR is used to verify clones using primers XZ-mgsA-up/ilvC-YZ347-down and sequenced, and a correct colony amplification product is 951 bp . A correct single colony is picked, and named as Sval014.

### Example 8: Integration of dihydroxy acid dehydratase encoding gene ilvD

Started from Sval014, a dihydroxy acid dehydratase encoding gene *ilvD* from *Escherichia coli* is integrated into the pyruvate formate lyase encoding gene *pflB* site and substitutes the *pflB* gene through a two-step homologous recombination method, namely the *pflB* gene is knocked out while the *ilvD* is integrated. Specific steps are as follows.

In a first step, a pXZ-CS plasmid DNA is used as a template, 2719 bp of a DNA fragment I is amplified by using primers pflB-CS-up/pflB-CS-down, and used for the first step of the homologous recombination. Amplification system and amplification conditions are the same as those described in Example 1. The DNA fragment I is electrotransformed into the Sval014.

The DNA fragment I is used for the first homologous recombination: firstly, a pKD46 plasmid is transformed into *Escherichia coli* Sval014 by an electrotransformation method, and then the DNA fragment I is electrotransformed into the *Escherichia coli* Sval014 with the pKD46.

Electrotransformation conditions and steps are the same as the first step method for the *mgsA* gene knockout described in Example 1. 200 µl of culture solution is spreaded onto a LB plate containing ampicillin (a final concentration is 100 µg/ml) and chloramphenicol (a final concentration is 34 µg/ml). After being cultured overnight at 30°C, colonies were PCR verified with primers XZ-pflB-up600/XZ-pflB-down, and a correct PCR product should be 3675 bp. A correct single colony is picked, and named as Sval015.

In a second step, a genomic DNA of *Escherichia coli* MG1655 (from ATCC, No. 700926) is used as a template, and 1951 bp of a DNA fragment II is amplified by using primers pflB-ilvD-up/pflB-ilvD-down. The DNA fragment II is used for the second homologous recombination. The DNA fragment II is electrotransformed into strain Sval015.

Electrotransformation conditions and steps are the same as the second step method for the *mgsA* gene knockout described in Example 1. Colonies were PCR verified using primers XZ-pflB-up600/XZ-pflB-down and sequenced, and a correct colony amplification product is 2907 bp .

A correct single colony is picked, and named as Sval016.

### Example 9: Regulation of dihydroxy acid dehydratase encoding gene ilvD

Started from Sval016, and an artificial regulatory element is used to regulate expression of the dihydroxy acid dehydratase encoding gene *ilvD* integrated in the pyruvate formate lyase encoding gene *pflB* site. Specific steps are as follows.

In a first step, a pXZ-CS plasmid DNA is used as a template, 2719 bp of a DNA fragment I is amplified by using primers pflB-Pcs-up/pflB-Pcs-down, and used for the first step of the homologous recombination. Amplification system and amplification conditions are the same as those described in Example 1. The DNA fragment I is electrotransformed into the Sval016.

The DNA fragment I is used for the first homologous recombination: firstly, a pKD46 plasmid is transformed into *Escherichia coli* Sval016 by an electrotransformation method, and then the DNA fragment I is electrotransformed into the *Escherichia coli* Sval016 with the pKD46.

Electrotransformation conditions and steps are the same as the first step method for the *mgsA* gene knockout described in Example 1. 200 µl of culture solution is spreaded onto a LB plate containing ampicillin (a final concentration is 100 µg/ml) and chloramphenicol (a final concentration is 34 µg/ml). After being cultured overnight at 30°C, colonies were PCR verified with primers XZ-pflB-up600/ilvD-YZ496-down, and a correct PCR product should be 3756 bp. A correct single colony is picked, and named as Sval017.

In a second step, a genomic DNA of M1-93 (Lu, et al., Appl Microbiol Biotechnol, 2012,93:2455-2462) is used as a template, and 189 bp of a DNA fragment II is amplified by using primers pflB-Pro-up/ilvD-Pro-down. The DNA fragment II is used for the second homologous recombination. The DNA fragment II is electrotransformed into strain Sval017.

Electrotransformation conditions and steps are the same as the second step method for the *mgsA* gene knockout described in Example 1. Colonies were PCR verified using primers XZ-pflB-up600/ilvD-YZ496-down and sequenced, and a correct colony amplification product is 1226 bp . A correct single colony is picked, and named as Sval018.

### Example 10: Regulation of acetolactate synthase gene ilvBN

An artificial regulatory element M1-93 is used to regulate expression of an acetolactate synthase gene *ilvBN* through a two-step homologous recombination method. Specific steps are as follows.

In a first step, a pXZ-CS plasmid DNA is used as a template, 2719 bp of a DNA fragment I is amplified by using primers ilvB pro-catup/ilvB pro-catdown, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval018 by an electrotransformation method, and then the DNA fragment I is electrotransformed into the *Escherichia coli* Sval018 with the pKD46.

Electrotransformation conditions and steps are the same as the first step method for the *mgsA* gene knockout described in Example 1. 200 µl of culture solution is spreaded onto a LB plate containing ampicillin (a final concentration is 100 µg/ml) and chloramphenicol (a final concentration is 34 µg/ml). After being cultured overnight at 30°C, colonies were PCR verified with primers ilvB pro-YZup/ilvB pro-YZdown, and a correct PCR product should be 2996 bp. A correct single colony is picked, and named as Sval019.

In a second step, a genomic DNA of M1-93 (Lu, et al., Appl Microbiol Biotechnol, 2012,93:2455-2462) is used as a template, and 188 bp of a DNA fragment II is amplified by using primers ilvB pro-up/ilvB pro-down. The DNA fragment II is used for the second homologous recombination. The DNA fragment II is electrotransformed into strain Sval019.

Electrotransformation conditions and steps are the same as the second step method for the *mgsA* gene knockout described in Example 1. Colonies were PCR verified using primers ilvB pro-YZup/ilvB pro-YZdown and sequenced, and a correct colony amplification product is 465 bp . A correct single colony is picked, and named as Sval020.

### Example 11: Regulation of acetolactate synthase gene ilvGM

An artificial regulatory element M1-93 is used to regulate expression of the acetolactate synthase gene *ilvGM* through a two-step homologous recombination method. Specific steps are as follows.

In a first step, a pXZ-CS plasmid DNA is used as a template, 2719 bp of a DNA fragment I is amplified by using primers ilvG pro-catup / ilvG pro-catdown, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval020 by an electrotransformation method, and then the DNA fragment I is electrotransformed into the *Escherichia coli* Sval020 with the pKD46.

Electrotransformation conditions and steps are the same as the first step method for the *mgsA* gene knockout described in Example 1. 200 µl of culture solution is spreaded onto a LB plate containing ampicillin (a final concentration is 100 µg/ml) and chloramphenicol (a final concentration is 34 µg/ml). After being cultured overnight at 30°C, colonies were PCR verified with primers ilvG pro-YZup / ilvG p-YZdown, and a correct PCR product should be 2993 bp. A correct single colony is picked, and named as Sval0121.

In a second step, a genomic DNA of M1-93 (Lu, et al., Appl Microbiol Biotechnol, 2012,93:2455-2462) is used as a template, and 188 bp of a DNA fragment II is amplified by using primers ilvG pro-up/ilvG pro-down. The DNA fragment II is used for the second homologous recombination. The DNA fragment II is electrotransformed into strain Sval021.

Electrotransformation conditions and steps are the same as the second step method for the *mgsA* gene knockout described in Example 1. Colonies were PCR verified using primers ilvG pro-YZup / ilvG p-YZdown and sequenced, and a correct colony amplification product is 462 bp . A correct single colony is picked, and named as Sval022.

### Example 12: Mutation of acetolactate synthase gene ilvH

A mutation is transferred into the *ilvH* gene so as to release feedback inhibition of L-valine through a two-step homologous recombination method. Specific steps are as follows.

In a first step, a pXZ-CS plasmid DNA is used as a template, 2719 bp of a DNA fragment I is amplified by using primers ilvH*-cat-up/ ilvH*-cat-down, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval022 by an electrotransformation method, and then the DNA fragment I is electrotransformed into the *Escherichia coli* Sval022 with the pKD46.

Electrotransformation conditions and steps are the same as the first step method for the *mgsA* gene knockout described in Example 1. 200 µl of culture solution is spreaded onto a LB plate containing ampicillin (a final concentration is 100 µg/ml) and chloramphenicol (a final concentration is 34 µg/ml). After being cultured overnight at 30°C, colonies were PCR verified using primers ilvH*-mutYZ-up/ ilvH*-mut-down, and a correct PCR product should be 3165 bp. A correct single colony is picked, and named as Sval023.

In a second step, a DNA of wild-type *Escherichia coli ATCC* 8739 is used as a template, and 467 bp of a DNA fragment II is amplified by using primers ilvH*-mut-up / ilvH*-mut-down. The DNA fragment

II is used for the second homologous recombination. The DNA fragment II is electrotransformed into strain Sval023.

Electrotransformation conditions and steps are the same as the second step method for the *mgsA* gene knockout described in Example 1. Colonies were PCR verified using primers ilvH*-mutYZ-up/ ilvH*-mut-down and sequenced, and a correct colony amplification product is 619 bp . A correct single colony is picked, and named as Sval024.

### Example 13: Fermentation and production of L-valine using recombinant strain Sval024

A seed culture medium is formed by the following components (a solvent is water):
Glucose 20 g/L, corn syrup dry powder 10 g/L, KH₂PO₄ 8.8 g/L, (NH₄)₂SO₄ 2.5 g/L, and MgSO₄
- 7H₂O 2 g/L.

The fermentation culture medium is most the same as the seed culture medium, and a difference is only that the glucose concentration is 50 g/L.

Anaerobic fermentation of Sval024 includes the following steps:
(1) Seed culture: a fresh clone on an LB plate is inoculated into a test tube containing 4 ml of the seed culture medium, and shake-cultured overnight at 37°C and 250 rpm. Then, a culture is transferred to 250 ml of a triangular flask containing 30 ml of the seed culture medium according to an inoculum size of 2% (V/V), and seed culture solution is obtained by shake culture at 37°C and 250 rpm for 12 hours, and used for fermentation medium inoculation.
(2) Fermentation culture: a volume of the fermentation culture medium in 500 ml of an fermenter is 250 ml, and the seed culture solution is inoculated into the fermentation culture medium according to an inoculum size of final concentration OD550=0.1, and fermented at 37°C and 150 rpm for 4 days, to obtain fermentation solution. The neutralizer is 5M ammonia, the pH was controlled at 7.0. No air was sparged during the fermentation.

Analytical method: an Agilent (Agilent-1260) high performance liquid chromatograph is used to determine components in the fermentation solution after fermentation for 4 days. The concentrations of glucose and organic acid in the fermentation solution are determined by using an Aminex HPX-87H organic acid analytical column of Biorad Company. A Sielc amino acid analysis column primesep 100 250x4.6mm is used for amino acid determination.

It is discovered from results that: strain Sval024 could produce 1.3 g/L of L-valine (L-valine peak corresponding to a position in Fig. 2 appears) with a yield of 0.31 mol/mol after 4 days fermentation under anaerobic conditions.

### Example 14: Cloning and integration of leucine dehydrogenase encoding gene leuDH

Referring to the reported (Ohshima, T. et.al, Properties of crystalline leucine dehydrogenase from Bacillus sphaericus. The Journal of biological chemistry 253, 5719-5725 (1978)) sequence of a leuDH from Lysinibacillus sphaericus IFO 3525, a leuDH gene was codon optimized and chemically synthesized (an optimized sequence is as shown in a sequence number 69). During the synthesis, an M1-93 artificial regulatory element is added before the *leuDH* gene to initiate expression of the *leuDH* gene, and inserted into a pUC57 vector to construct a plasmid pUC57-M1-93-leuDH (gene synthesis and vector construction are completed by Nanjing Genscript Biotechnology Co., Ltd.). The M1-93 artificial regulatory element and the *leuDH* gene are integrated into the fumarate reductase encoding gene *frd* site in strain Sval024 through a two-step homologous recombination method and substitute the *frd* gene, namely the *frd* gene is knocked out while the *leuDH* is integrated. Specific steps are as follows.

In a first step, a pXZ-CS plasmid DNA is used as a template, 2719 bp of a DNA fragment I is amplified by using primers frd-cs-up / frd-cs-down, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval024 by an electrotransformation method, and then the DNA fragment I is electrotransformed into the *Escherichia coli* Sval024 with the pKD46.

Electrotransformation conditions and steps are the same as the first step method for the *mgsA* gene knockout described in Example 1. 200 µl of culture solution is spreaded onto a LB plate containing ampicillin (a final concentration is 100 µg/ml) and chloramphenicol (a final concentration is 34 µg/ml). After being cultured overnight at 30°C, colonies were PCR verified with primers XZ-frd-up/XZ-frd-down, and a correct PCR product should be 3493 bp. A correct single colony is picked, and named as Sval025.

In a second step, a pUC57-M1 -93-leuDH plasmid DNA is used as a template, and 1283 bp of a DNA fragment II is amplified by using primers frd-M93-up/frd-leuDH-down. The DNA fragment II is used for the second homologous recombination. The DNA fragment II is electrotransformed into strain Sval025.

Electrotransformation conditions and steps are the same as the second step method for the *mgsA* gene knockout described in Example 1. Colonies PCR is used to verify clones using primers XZ-frd-up/XZ-frd-down and sequenced, and a correct colony amplification product is 2057 bp . A correct single colony is picked, and named as Sval026.

### Example 15: Fermentation and production of L-valine using recombinant strain Sval026

Components and preparation of seed culture medium and fermentation culture medium are the same as those described in Example 13.

The fermentation is performed in 500 mL of a fermentation vessel, and a fermentationvessel and an analysis process are the same as the fermentation process and the analysis process of the Sval024 described in Example 13.

It is discovered from results that: the strain Sval026 could produce 1.8 g/L of L-valine (L-valine peak corresponding to a position in Fig. 2 appears) 0.56 mol/mol after 4 days fermentation under anaerobic conditions.

### Example 16: Integration of NADH-dependent acetohydroxy acid reductoisomerase encoding gene in alcohol dehydrogenase gene adhEsite

An acetohydroxy acid reductoisomerase encoding gene *kari* is obtained according to a *kari* sequence from a *Thermacetogenium phaeum* strain reported in a literature (Brinkmann-Chen, S., Cahn, JKB & Arnold, FH Uncovering rare NADH-preferring ketol-acid reductoisomerases. Metab Eng 26, 17-22, doi: 10.1016 /j.ymben.2014.08.003 (2014).) and through whole gene synthesis after being codon-optimized (an optimized sequence refers to a sequence 70). During the synthesis, the RBS5 artificial regulatory element is added in front of the *kari* gene and used to initiate expression of the *kari* gene, and inserted in the pUC57 vector, so a plasmid pUC57-RBS5-kari (gene synthesis and vector construction are completed by Nanjing Genscript Biotechnology Co., Ltd.) is constructed and obtained. The RBS5 artificial regulatory element and the *kari* gene are integrated into the *adhE* site of the alcohol dehydrogenase encoding gene in the strain Sval026 through a two-step homologous recombination method. Specific steps are as follows.

In a first step, a *cat-sacB* gene is integrated into the *adhE* gene site in the Sval026, acquisition and purification of a fragment, integration of the first homologous recombination, and verification are the exactly same as the fragment and method used for the first step of the homologous recombination in the *adhE* gene knockout in Example 5, and clones obtained is named as Sval061 (Table 1).

In a second step, a pUC57-RBS5-kari plasmid DNA is used as a template, 1188 bp of a DNA fragment II is amplified with primers adhE-RBS5-up/adhE-kari-down. The DNA fragment II is used for the second homologous recombination. The DNA fragment II is electrotransformed into strain Sval061.

Electroporation conditions and steps are the same as the second step method for the *mgsA* gene knockout described in Example 1. Colonies were verified by PCR using primers XZ-adhE-up/XZ-adhE-down and sequenced, and a correct colony amplification product is 1636 bp . A correct single colony is picked, and named as Sval062.

### Example 17: Knockout of NADPH-dependent acetohydroxy acid reductoisomerase encoding gene in mgsA site

An NADPH-dependent acetohydroxy acid reductoisomerase encoding gene *ilvC* integrated in an *mgsA* site of a methylglyoxal synthase encoding gene is knocked out through a two-step homologous recombination method. Specific steps are as follows.

In a first step, a cat-sacB gene is integrated into the *mgsA* site to substitute the *ilvC* gene, acquisition and purification of a fragment, integration of the first homologous recombination, and verification are the exactly same as the fragment and method used for the first step of the homologous recombination in the *mgsA* gene knockout in Example 1, and a clone obtained is named as Sval063 (Table 1).

In a second step, the cat-sacB fragment is substituted with the fragment knocked out by using the *mgsA,* to obtain a strain knocked out by the *mgsA* gene and *ilvC* gene. Acquisition and purification of a fragment, integration of the second homologous recombination, and verification are the exactly same as the fragment and method used for the second step of the homologous recombination in the *mgsA* gene knockout in Example 1, and a clone obtained is named as Sval064.

### Example 18: Production of L-valine using recombinant strain Sval064

Components and preparation of seed culture medium and fermentation culture medium are the same as those described in Example 13.

The fermentation is performed in 500 mL of a fermentation vessel, a fermentation process and an analysis process are the same as the fermentation process and the analysis process of the Sval024 described in Example 13.

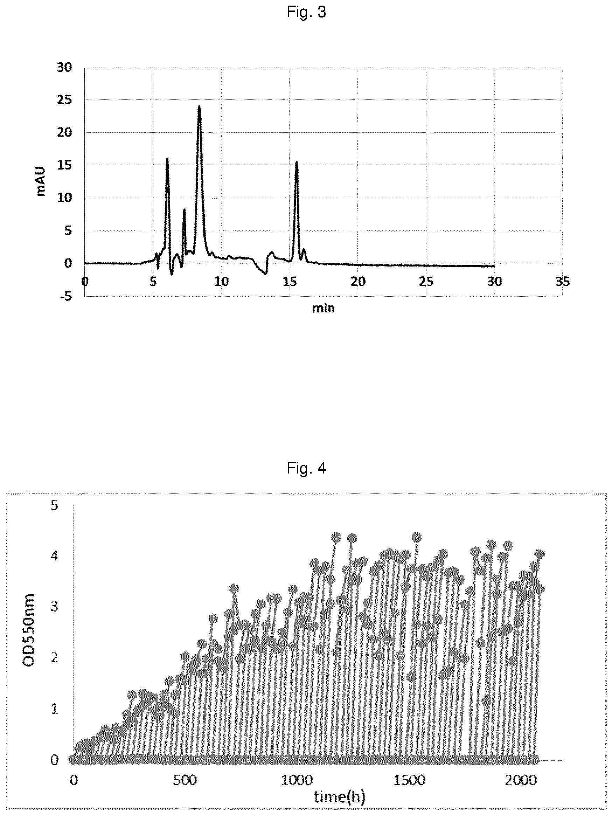

It is discovered from results that: the strain Sval064 could produce 2.0 g/L of L-valine (L-valine peak corresponding to a position in Fig. 2 appears) with a yield of 0.80 mol/mol (Fig. 3) after fermentation for 4 days under anaerobic conditions.

### Example 19: Construction of recombinant strain Sval065

Started from Sval064, cell growth and L-valine production capacity are synchronously improved through metabolic evolution.

Metabolic evolution was carried out in 500 mL fermentation vessel with 250 mL fermentation culture medium. The fermentative pH was controlled at 7.0 by using 5 Mammonia as neutralizer. Components and preparation method of fermentation culture medium used for the metabolic evolution are the same as those of the fermentation culture medium described in Example 16. Every 24 hours, fermentation solution is transferred into a new fermentation vessel and the initial OD550 is 0.1. After 105 generations of the evolution, a strain Sval065 is obtained (Fig. 4). The strain Sval065 is preserved in China General Microbiological Culture Collection Center (CGMCC) with the deposit number CGMCC 19458.

### Example 20: Fermentation of strain Sval065 to produce L-valine in 500 mL fermentation vessel

Components and preparation of a seed culture medium are the same as those described in Example 13.

The fermentation is performed in 500 mL of a fermentation vessel, and a fermentation culture medium is 250 ml. The fermentation culture medium is basically the same as the seed culture medium. A difference is that a glucose concentration is 100 g/L, and a neutralizer used is 5M ammonia, so that fermentative pH is controlled in 7.0.

It is discovered from results that: after fermented for 48 hours, strain Sval065 produced 45 g/LL-valine with a yield of 0.9 mol/mol, and impurities such as a heteroacid are not generated.

### Example 21: Production of L-valine by fermentation of recombinant strain Sval065 in 5 L fermentation vessel

Components, preparation and analytical method of a seed culture medium are the same as those described in Example 13. A fermentation culture medium is basically the same as the seed culture medium, and a difference is that a glucose concentration is 140 g/L.

The fermentation is performed in 5 L of a fermentation vessel (Shanghai Baoxing, BIOTECH-5BG) under anaerobic conditions, including the following steps:
(1) Seed culture: the seed culture medium in 500 ml of a triangular flask is 150 ml, and it is sterilized at 115°C for 15 min. After cooling, recombinant *Escherichia coli* Sval045 are inoculated into the seed culture medium according to an inoculum size of 1% (V/V), and cultured at 37°C and 100 rpm for 12 hours to obtain seed solution for inoculation of the fermentation culture medium.
(2) Fermentation culture: a volume of the fermentation culture medium in 5 L is 3 L, and it is sterilized at 115°C for 25 min. The seed solution is inoculated into the fermentation culture medium according to an inoculum size of final concentration OD550=0.2, and cultured under anaerobic conditions at 37°C for 3 days, and a stirring speed is 200 rpm, fermentation solution is obtained. The fermentation solution is all of substances in the fermentation vessel. No air was sparged during the fermentation.

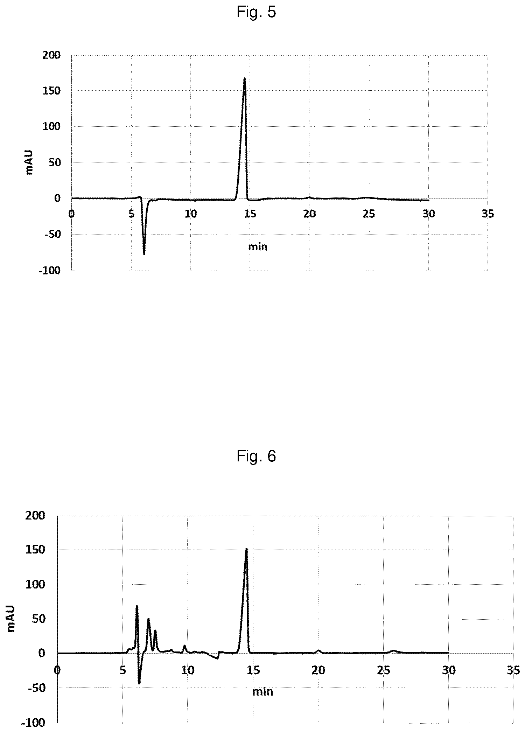

It is discovered from results that: after fermented for 48 hours, strain Sval065 produced 83 g/L L-valine with a yield of0.92 mol/mol (0.6 g/g), and impurities such as a heteroacid are not generated (Figs. 5-6).

## Claims

1. A construction method of a recombinant microorganism for producing L-valine comprising:
transferring an acetohydroxy acid isoreductase gene and/or an amino acid dehydrogenase gene into a microorganism, so that enhancing enzymatic activity of the acetohydroxy acid isoreductase gene and/or the amino acid dehydrogenase;
preferably, the acetohydroxy acid isoreductase gene and/or the amino acid dehydrogenase gene are NADH-dependent;
preferably, the acetohydroxy acid isoreductase gene is *ilvC* or *KARI;* and the amino acid dehydrogenase gene is a leucine dehydrogenase gene; and
more preferably, the acetohydroxy acid isoreductase gene is *KARI,* and the leucine dehydrogenase gene is *leuDH.*

2. The construction method according to claim 1, wherein the method further comprises one or more of the following modifications (1)-(7) to the recombinant microorganism according to claim 1:
(1) knocking out a gene *mgsA;*
(2) knocking out a gene *IdhA*;
(3) knocking out genes *pta* and/or *ackA;*
(4) knocking out genes *tdcD* and/or *tdcE;*
(5) knocking out a gene *adhE;*
(6) knocking out genes *frd* and/or *pflB;* and
(7) enhancing activity of acetolactate synthase AHAS and/or dihydroxy acid dehydratase *ilvD;* preferably, the AHAS is ilvBN, or ilvGM, or ilvlH; optionally, the activity of the ilvlH AHAS is enhanced by releasing feedback inhibition of valine on the ilvH, preferably, the *ilvH* gene is enhanced by mutation;
preferably, the above item (7) is selected for modification;
preferably, the above items (7) and (2) are selected for modification;
preferably, the above items (7) and (6) are selected for modification;
preferably, the above items (7), (2) and (5) are selected for modification;
preferably, the above items (7), (1), and (3)-(6) are selected for modification;
preferably, the above items (1)-(7) are selected for modification;
Preferably, releasing the feedback inhibition of valine to ilvH by *ilvH* gene mutation;
preferably, the item (6) is achieved by substituting the *pflB* gene of the microorganism itself with the *ilvD* gene;
preferably, the item (6) is achieved by substituting the *frd* gene of the microorganism itself with the *leuDH* gene;
preferably, the item (5) is achieved by substituting the *adHE* gene of the microorganism itself with the *KARI* gene; and
preferably, the item (1) is achieved by substituting the *mgsA* gene of the microorganism itself with the *ilvC* gene, and the *ilvC* is optionally knocked out again after being transferred.

3. The construction method according to claim 1 or 2, wherein the microorganism is *Escherichia coli;* and more preferably, the microorganism is *Escherichia coli ATCC* 8739.

4. The construction method according to any one of claims 1 to 3, wherein at least one regulatory element is used to enhance activity of a gene of the enzyme;
preferably, the regulatory element is selected from an M1-46 artificial regulatory element, an M1-93 artificial regulatory element or a RBS5 artificial regulatory element;
preferably, the M1-46 artificial regulatory element regulates an encoding gene *ilvC;*
the M1-93 artificial regulatory element regulates *ilvD, leuDH, ilvBN and ilvGM* genes; and
the RBS5 artificial regulatory element regulates a *KARl* gene.

5. The construction method according to any one of claims 1-4, wherein one or more copies of the enzyme encoding gene and the regulatory element are integrated into a genome of the microorganism, or a plasmid containing the enzyme encoding gene is transferred into the microorganism;
preferably, transfer, mutation, knockout or regulation of the enzyme gene is completed by a method of integrating into the genome of the microorganism;
preferably, the transfer, mutation, knockout or regulation of the enzyme gene is completed by homologous recombination method; and
preferably, the transfer, mutation, knockout or regulation of the enzyme gene is completed by a two-step homologous recombination method.

6. A recombinant microorganism obtained by the construction method according to any one of claims 1-5.

7. A method for acquiring a recombinant microorganism for highly producing L-valine, wherein it is obtained through metabolic evolution on the basis of the recombinant microorganism according to claim 5.

8. A recombinant microorganism, wherein the deposit number thereof is CGMCC 19458.

9. Use of the recombinant microorganism according to claim 6 or claim 8 in production of L-valine.

10. A method for producing L-valine, wherein the method comprises: (1) fermenting the recombinant microorganism according to claim 6 or 8; and (2) separating and harvesting L-valine; preferably, the fermentation is carried out under anaerobic conditions.


SEQUENCE LISTING

<110>  Anhui Huaheng Biological Engineering Co., Ltd.
       Tianjin Institute of Industrial Biotechnology, Chinese Academy of
Sciences

<120>  Recombinant microorganism for producing L-valine, construction method
and application thereof

<130>  PN151382HHSW

<150>  CN202010401422.5
<151>  2020-05-13

<160>  70

<170>  PatentIn version 3.5

<210>  1
<211>  73
<212>  DNA
<213>  Escherichia coli

<400>  1
gtaggaaagt taactacgga tgtacattat ggaactgacg actcgcactt tgtgacggaa      60

gatcacttcg cag                                                          73


<210>  2
<211>  75
<212>  DNA
<213>  Escherichia coli

<400>  2
gcgtttgcca cctgtgcaat attacttcag acggtccgcg agataacgct ttatttgtta      60

actgttaatt gtcct                                                        75


<210>  3
<211>  20
<212>  DNA
<213>  Escherichia coli

<400>  3
cagctcatca accaggtcaa                                                   20


<210>  4
<211>  20
<212>  DNA
<213>  Escherichia coli

<400>  4
aaaagccgtc acgttattgg                                                   20


<210>  5
<211>  70
<212>  DNA
<213>  Escherichia coli

<400>  5
gcgtttgcca cctgtgcaat attacttcag acggtccgcg agataacgct aagtgcgagt      60

cgtcagttcc                                                              70


<210>  6
<211>  72
<212>  DNA
<213>  Escherichia coli

<400>  6
gtaggaaagt taactacgga tgtacattat ggaactgacg actcgcactt atggctaact      60

acttcaatac ac                                                           72


<210>  7
<211>  75
<212>  DNA
<213>  Escherichia coli

<400>  7
gcgtttgcca cctgtgcaat attacttcag acggtccgcg agataacgct ttaacccgca      60

acagcaatac gtttc                                                        75


<210>  8
<211>  73
<212>  DNA
<213>  Escherichia coli

<400>  8
gtaggaaagt taactacgga tgtacattat ggaactgacg actcgcactt tgtgacggaa      60

gatcacttcg cag                                                          73


<210>  9
<211>  75
<212>  DNA
<213>  Escherichia coli

<400>  9
agctgtgcca gctgctggcg cagattcagt gtattgaagt agttagccat ttatttgtta      60

actgttaatt gtcct                                                        75


<210>  10
<211>  70
<212>  DNA
<213>  Escherichia coli

<400>  10
gtaggaaagt taactacgga tgtacattat ggaactgacg actcgcactt ttatctctgg      60

cggtgttgac                                                              70


<210>  11
<211>  72

<212>  DNA
<213>  Escherichia coli

<400>  11
agctgtgcca gctgctggcg cagattcagt gtattgaagt agttagccat agctgtttcc      60

tggtttaaac cg                                                          72


<210>  12
<211>  20
<212>  DNA
<213>  Escherichia coli

<400>  12
cgcactacat cagagtgctg                                                  20


<210>  13
<211>  73
<212>  DNA
<213>  Escherichia coli

<400>  13
ttcaacatca ctggagaaag tcttatgaaa ctcgccgttt atagcacaaa tgtgacggaa      60

gatcacttcg cag                                                         73


<210>  14
<211>  75
<212>  DNA
<213>  Escherichia coli

<400>  14
agcggcaaga ttaaaccagt tcgttcgggc aggtttcgcc tttttccaga ttatttgtta      60

actgttaatt gtcct                                                       75


<210>  15
<211>  20
<212>  DNA
<213>  Escherichia coli

<400>  15
gataacggag atcgggaatg                                                  20


<210>  16
<211>  20
<212>  DNA
<213>  Escherichia coli

<400>  16
ctttggctgt cagttcacca                                                  20


<210>  17
<211>  71
<212>  DNA
<213>  Escherichia coli

<400>  17
agcggcaaga ttaaaccagt tcgttcgggc aggtttcgcc tttttccaga tttgtgctat      60

aaacggcgag t                                                           71


<210>  18
<211>  73
<212>  DNA
<213>  Escherichia coli

<400>  18
aggtacttcc atgtcgagta agttagtact ggttctgaac tgcggtagtt tgtgacggaa      60

gatcacttcg cag                                                         73


<210>  19
<211>  75
<212>  DNA
<213>  Escherichia coli

<400>  19
ggtcggcaga acgctgtacc gctttgtagg tggtgttacc ggtgttcaga ttatttgtta      60

actgttaatt gtcct                                                       75


<210>  20
<211>  20
<212>  DNA
<213>  Escherichia coli

<400>  20
cgggacaacg ttcaaaacat                                                  20


<210>  21
<211>  20
<212>  DNA
<213>  Escherichia coli

<400>  21
attgcccatc ttcttgttgg                                                  20


<210>  22
<211>  71
<212>  DNA
<213>  Escherichia coli

<400>  22
ggtcggcaga acgctgtacc gctttgtagg tggtgttacc ggtgttcaga aactaccgca      60

gttcagaacc a                                                           71


<210>  23
<211>  73
<212>  DNA
<213>  Escherichia coli

<400>  23

ccgtgattgg tctgctgacc atcctgaaca tcgtatacaa actgttttaa tgtgacggaa 60

gatcacttcg cag 73

<210> 24
<211> 75
<212> DNA
<213> Escherichia coli

<400> 24
cgcctggggc acgttgcgtt tcgataatct ttttcataca tcctccggcg ttatttgtta 60

actgttaatt gtcct 75

<210> 25
<211> 21
<212> DNA
<213> Escherichia coli

<400> 25
tgatgagcta cctggtatgg c 21

<210> 26
<211> 24
<212> DNA
<213> Escherichia coli

<400> 26
cgccgacaga gtaataggtt ttac 24

<210> 27
<211> 77
<212> DNA
<213> Escherichia coli

<400> 27
cgcctggggc acgttgcgtt tcgataatct ttttcataca tcctccggcg ttaaaacagt 60

ttgtatacga tgttcag 77

<210> 28
<211> 72
<212> DNA
<213> Escherichia coli

<400> 28
ataactctaa tgtttaaact cttttagtaa atcacagtga gtgtgagcgc tgtgacggaa 60

gatcacttcg ca 72

<210> 29
<211> 75
<212> DNA
<213> Escherichia coli

<400> 29
ccgtttatgt tgccagacag cgctactgat taagcggatt ttttcgcttt ttatttgtta 60

actgttaatt gtcct                                                    75

<210>  30
<211>  74
<212>  DNA
<213>  Escherichia coli

<400>  30
ccgtttatgt tgccagacag cgctactgat taagcggatt ttttcgcttt gcgctcacac      60

tcactgtgat ttac                                                    74

<210>  31
<211>  21
<212>  DNA
<213>  Escherichia coli

<400>  31
catgctaatg tagccaccaa a                                            21

<210>  32
<211>  20
<212>  DNA
<213>  Escherichia coli

<400>  32
ttgcaccacc atccagataa                                              20

<210>  33
<211>  70
<212>  DNA
<213>  Escherichia coli

<400>  33
ataactctaa tgtttaaact cttttagtaa atcacagtga gtgtgagcgc ttatctctgg      60

cggtgttgac                                                         70

<210>  34
<211>  70
<212>  DNA
<213>  Escherichia coli

<400>  34
ccgtttatgt tgccagacag cgctactgat taagcggatt ttttcgcttt ttagataact      60

tttttcttca                                                         70

<210>  35
<211>  73
<212>  DNA
<213>  Escherichia coli

<400>  35
aaacgaccac cattaatggt tgtcgaagta cgcagtaaat aaaaaatcca tgtgacggaa      60

gatcacttcg cag                                                          73

<210>  36
<211>  75
<212>  DNA
<213>  Escherichia coli

<400>  36
cggtccgaac ggcgcgccag cacgacgacc gtctggggtg ttacccgttt ttatttgtta      60

actgttaatt gtcct                                                       75

<210>  37
<211>  70
<212>  DNA
<213>  Escherichia coli

<400>  37
aaacgaccac cattaatggt tgtcgaagta cgcagtaaat aaaaaatcca atgcctaagt      60

accgttccgc                                                             70

<210>  38
<211>  74
<212>  DNA
<213>  Escherichia coli

<400>  38
cggtccgaac ggcgcgccag cacgacgacc gtctggggtg ttacccgttt ttaacccccc      60

agtttcgatt tatc                                                        74

<210>  39
<211>  21
<212>  DNA
<213>  Escherichia coli

<400>  39
ctgcggagcc gatctcttta c                                                21

<210>  40
<211>  22
<212>  DNA
<213>  Escherichia coli

<400>  40
cgagtaataa cgtcctgctg ct                                               22

<210>  41
<211>  72
<212>  DNA
<213>  Escherichia coli

<400>  41
aaacgaccac cattaatggt tgtcgaagta cgcagtaaat aaaaaatcca tgtgacggaa      60

gatcacttcg ca                                                          72

<210>  42
<211>  75
<212>  DNA
<213>  Escherichia coli

<400>  42
cccgccatat tacgaccatg agtggtggtg gcggaacggt acttaggcat ttatttgtta      60

actgttaatt gtcct                                                        75


<210>  43
<211>  70
<212>  DNA
<213>  Escherichia coli

<400>  43
aaacgaccac cattaatggt tgtcgaagta cgcagtaaat aaaaaatcca ttatctctgg      60

cggtgttgac                                                              70


<210>  44
<211>  78
<212>  DNA
<213>  Escherichia coli

<400>  44
cccgccatat tacgaccatg agtggtggtg gcggaacggt acttaggcat tgctgacctc      60

ctggtttaaa cgtacatg                                                     78


<210>  45
<211>  20
<212>  DNA
<213>  Escherichia coli

<400>  45
caaccagatc gagcttgatg                                                   20


<210>  46
<211>  20
<212>  DNA
<213>  Escherichia coli

<400>  46
tgcagaaaac catcgacaag                                                   20


<210>  47
<211>  20
<212>  DNA
<213>  Escherichia coli

<400>  47
caccaatcag cgtgacaact                                                   20


<210>  48

<211>  72
<212>  DNA
<213>  Escherichia coli

<400>  48
gaaggcgaat ggctgagatg aaaaacctga aaattgaggt ggtgcgctat tgtgacggaa      60

gatcacttcg ca                                                          72


<210>  49
<211>  75
<212>  DNA
<213>  Escherichia coli

<400>  49
tctcaggctc cttaccagta cagggcaaca aacaggatta cgatggtggc ttatttgtta      60

actgttaatt gtcct                                                       75


<210>  50
<211>  70
<212>  DNA
<213>  Escherichia coli

<400>  50
gaaggcgaat ggctgagatg aaaaacctga aaattgaggt ggtgcgctat ttatctctgg      60

cggtgttgac                                                             70


<210>  51
<211>  77
<212>  DNA
<213>  Escherichia coli

<400>  51
tctcaggctc cttaccagta cagggcaaca aacaggatta cgatggtggc ttaacggccg      60

ttcaaaatat ttttttc                                                     77


<210>  52
<211>  72
<212>  DNA
<213>  Escherichia coli

<400>  52
ctgacgaaac ctcgctccgg cggggttttt tgttatctgc aattcagtac tgtgacggaa      60

gatcacttcg ca                                                          72


<210>  53
<211>  75
<212>  DNA
<213>  Escherichia coli

<400>  53
tctgcgccgg taaagcgctt acgcgtcgat gttgtgcccg aacttgccat ttatttgtta      60

actgttaatt gtcct                                                       75

<210>  54
<211>  70
<212>  DNA
<213>  Escherichia coli

<400>  54
ctgacgaaac ctcgctccgg cggggttttt tgttatctgc aattcagtac ttatctctgg      60

cggtgttgac                                                              70


<210>  55
<211>  70
<212>  DNA
<213>  Escherichia coli

<400>  55
tctgcgccgg taaagcgctt acgcgtcgat gttgtgcccg aacttgccat agctgtttcc      60

tggtttaaac                                                              70


<210>  56
<211>  22
<212>  DNA
<213>  Escherichia coli

<400>  56
gttctgcgcg gaacacgtat ac                                                22


<210>  57
<211>  20
<212>  DNA
<213>  Escherichia coli

<400>  57
ccgctacagg ccatacagac                                                   20


<210>  58
<211>  72
<212>  DNA
<213>  Escherichia coli

<400>  58
tgaactaaga ggaagggaac aacattcaga ccgaaattga atttttttca tgtgacggaa      60

gatcacttcg ca                                                           72


<210>  59
<211>  75
<212>  DNA
<213>  Escherichia coli

<400>  59
ttcacaccct gtgcccgcaa cgcatgtacc acccactgtg cgccattcat ttatttgtta      60

actgttaatt gtcct                                                        75

<210>  60
<211>  70
<212>  DNA
<213>  Escherichia coli

<400>  60
tgaactaaga ggaagggaac aacattcaga ccgaaattga atttttttca ttatctctgg        60

cggtgttgac                                                               70


<210>  61
<211>  71
<212>  DNA
<213>  Escherichia coli

<400>  61
ttcacaccct gtgcccgcaa cgcatgtacc acccactgtg cgccattcat agctgtttcc        60

tggtttaaac g                                                             71


<210>  62
<211>  22
<212>  DNA
<213>  Escherichia coli

<400>  62
gcataagata tcgctgctgt ag                                                 22


<210>  63
<211>  20
<212>  DNA
<213>  Escherichia coli

<400>  63
gccagttttg ccagtagcac                                                    20


<210>  64
<211>  72
<212>  DNA
<213>  Escherichia coli

<400>  64
agaacctgat tatgcgccgg atattatcag tcttactcga aaatgaatca tgtgacggaa        60

gatcacttcg ca                                                            72


<210>  65
<211>  75
<212>  DNA
<213>  Escherichia coli

<400>  65
ttcatcgccc acggtctgga tggtcatacg cgataatgtc ggatcgtcgg ttatttgtta        60

actgttaatt gtcct                                                         75

<210>  66
<211>  74
<212>  DNA
<213>  Escherichia coli

<400>  66
agaacctgat tatgcgccgg atattatcag tcttactcga aaatgaatca gacgcgttat      60

tccgcgtgat tggc                                                        74


<210>  67
<211>  21
<212>  DNA
<213>  Escherichia coli

<400>  67
cacaccagag cgagcaacct c                                                21


<210>  68
<211>  23
<212>  DNA
<213>  Escherichia coli

<400>  68
atgagctgga aagcaaactt agc                                              23


<210>  69
<211>  1095
<212>  DNA
<213>  Lysinibacillus sphaericus

<400>  69
atggaaatct tcaagtatat ggaaaagtat gattatgaac aattggtatt ttgccaagac      60

gaagcatctg ggttaaaagc gattatcgct atccatgaca caacacttgg accagcatta     120

ggtggtgctc gtatgtggac ctacgcgaca gaagaaaatg cgattgagga tgcattaaga     180

ttagcacgcg ggatgacata taaaaatgca gctgctggtt taaaccttgg cggtggaaaa     240

acggtcatta ttggggaccc atttaaagat aaaaacgaag aaatgttccg tgctttaggt     300

cgtttcattc aaggattaaa cggtcgctat attaccgctg aagatgttgg tacaaccgta     360

acagatatgg atttaatcca tgaggaaaca aattacgtta caggtatatc gccagcgttt     420

ggttcatcgg gtaatccttc accagtaact gcttatggcg tttatcgtgg catgaaagca     480

gcggcgaaag aagcatttgg tacggatatg ctagaaggtc gtactatatc ggtacaaggg     540

ctaggaaacg tagcttacaa gctttgcgag tatttacata atgaaggtgc aaaacttgta     600

gtaacagata ttaaccaagc ggctattgat cgtgttgtca atgattttgg cgctacagca     660

gttgcacctg atgaaatcta ttcacaagaa gtcgatattt tctcaccgtg tgcacttggc     720

gcaattttaa atgacgaaac gattccgcaa ttaaaagcaa aagttattgc tggttctgct     780

aataaccaac tacaagattc acgacatgga gattatttac acgagctagg cattgtttat     840

gcacctgact atgtcattaa tgcaggtggt gtaataaatg tcgcggacga attatatggc 900

tataatcgtg aacgagcgtt gaaacgtgta gatggtattt acgatagtat tgaaaaaatc 960

tttgaaattt ccaaacgtga tagtattcca acatatgttg cggcaaatcg tttggcagaa 1020

gaacgtattg ctcgtgtagc gaaatcgcgt agtcagttct taaaaaatga aaaaaatatt 1080

ttgaacggcc gttaa 1095

<210> 70
<211> 999
<212> DNA
<213> Thermacetogenium phaeum

<400> 70
atgaaaatct attacgatca ggacgcggat ctgcaatatc tggatggcaa aaccgtggct 60

gttatcggct acggttcaca gggccatgcg cagtcgcaaa atctgcgtga cagcggtgtt 120

aaagtggttg tcgcggatat tccgagctct gaaaactgga aaaaagctga agaagcgcag 180

ttccaaccgc tgacggctga cgaagcagcc cgcgaagcgg atattatcca gattctggtg 240

ccggatgaaa aacaagcagc tctgtatcgt gaatcaatcg ccccgaatct gcgcccgggc 300

aaagcactgg tgtttagcca cggcttcaac attcacttta aacagatcgt gccgccgccg 360

gacgtcgatg tgtttatggt cgcaccgaaa ggtccgggtc acctggtgcg tcgcatgtac 420

gaagaaggcg ccggtgttcc gtctctggtt gcagtcgaac aggactatag tggtcaagcc 480

ctgaatctgg cgctggccta cgcaaaaggc attggtgcca cccgtgcagg cgtcatccag 540

accacgttca aagaagaaac cgaaaccgac ctgtttggtg aacaagccgt cctgtgcggc 600

ggtattaccg aactgatccg cgcaggcttc gacaccctgg tggatgctgg ttatcagccg 660

gaaattgcgt actttgaatg tctgcatgaa atgaaactga ttgttgacct gatctatgaa 720

ggcggtattt ccaccatgcg ttatagtatc tccgacaccg ctgaatacgg cgatctgacg 780

cgtggtaaac gcattatcac cgaagcgacg cgcgaagaaa tgaagaaaat tctgaaagaa 840

atccaggatg gcgtgttcgc ccgtgaatgg ctgctggaaa accaagtggg tcgcccggtt 900

tataatgccc tgcgtcgcaa agaacagaac cacctgattg aaaccgtggg cgcacgtctg 960

cgcggtatga tgccgtggct gaagaaaaaa gttatctaa 999